# EUROPEAN PATENT APPLICATION

(11) **EP 2 839 778 A1**
(43) Date of publication of application: **25.02.2015**
(21) Application number: 14181585.2
(22) Date of filing: 20.08.2014
(51) Int. Cl.: A61B 5/021, A61B 5/022, A61B 5/024, A61B 5/00

(54) **Pulse wave measuring apparatus**

(30) Priority: 22.08.2013 JP 2013172398
(71) Applicant: Seiko Epson Corporation, Tokyo 163-0811 (JP)
(72) Inventor: Narusawa, Atsushi, Nagano, 392-8502 (JP)
(74) Representative: Hoffmann Eitle

(57) **Abstract**

A first light receiving unit is disposed so as to be separated at a first distance along a contact surface of the living body from a light emitting unit that comes into contact with a living body and radiates light wi th a determined wavelength toward the inside of the living body, presses a first site of the living body at a determined pressure, and receives the light reflected from the living body through the first site. A second light receiving unit is disposed so as to be separated at a second distance along the contact surface of the living body that is longer than the first distance from the light emitting unit that comes into contact with the living body and radiates light with the wavelength toward the inside of the living body, presses a second site that is different from the first site of the living body at a determined pressure, and receives the light reflected from the living body through the second site. A measuring unit measures the pulse wave of the living body based on respective signals that represent light received by the first light receiving unit and light received by the second light receiving unit.

## Description

### BACKGROUND

### 1. Technical Field

The present invention relates to an apparatus of measuring a pulse wave of a living body.

### 2. Related Art

Since a pulse wave appears as a change of volume of blood, it is possible to measure the pulse wave using a photoelectric pulse wave sensor that detects a change in blood volume of a site which becomes a subject to be measured. However, the volume of blood also changes depending on a movement of a human body (hereinafter, referred to as body movement) in addition to a heartbeat (that is, a pulse wave). For this reason, when measuring the pulse wave using the photoelectric pulse wave sensor, there is a case where noise caused by the body movement is included in a wave motion in the process of transmission from the heart to a site to be detected. That is, since blood is a fluid and the blood vessels are elastic, there is a case where the flow of blood generated by the body movement causes a change in an amount of blood, the flow being measured as a pseudo-pulsation.

A pulse wave measuring apparatus that performs an operation for removing a noise component due to such a body movement has been developed. For example, JP-A-55-120858 discloses a method of radiating light beams having different wavelengths, simultaneously measuring reflected light beams thereof, and extracting pulsation components from the measured values. The method uses the fact that there are different light absorption characteristics between oxygenated hemoglobin which is dominant in arterial blood and reduced hemoglobin which is dominant in venous blood.

However, the irradiation light beams that have different wavelengths and are used in a sensor which detects the reflected light beams and measures the pulse wave also have different penetration depths of light inside of a living body. For this reason, in the technology disclosed in JP-A-55-120858, the differences of light absorbance occurring among a plurality of sensors also include an influence caused by the differences of penetration depths of light having different wavelengths, and therefore, it is difficult to remove the noise caused by the body movement.

### SUMMARY

An advantage of some aspects of the invention is to provide a technology of removing noise from a plurality of signals including pulse waves without depending on the difference of a wavelength of light to be irradiated.

An aspect of the invention is directed to a pulse wave measuring apparatus including: a first light receiving unit which is disposed so as to be separated at a first distance along a contact surface of the living body from a light emitting unit that comes into contact with a living body and radiates light with a determined wavelength toward the inside of the living body, presses a first site of the living body at a determined pressure, and receives the light reflected from the living body through the first site; a second light receiving unit which is disposed so as to be separated at a second distance along the contact surface of the living body that is longer than the first distance from the light emitting unit that comes into contact with the living body and radiates light with the wavelength toward the inside of the living body, presses a second site that is different from the first site of the living body at the pressure, and receives the light reflected from the living body through the second site; and a measuring unit that measures the pulse wave of the living body based on respective signals that represent light received by the first light receiving unit and light received by the second light receiving unit.

According to this configuration, it is possible to remove noise from a plurality of signals including pulse waves without depending on the difference of a wavelength of light to be irradiated.

In the aspect of the invention described above, it is preferable that the first distance is from 2 mm to 4 mm.

In the aspect of the invention described above, it is preferable that the second distance is from 4 mm to 10 mm.

According to this configuration, it is easy to measure the two types of signals that represent the pulse wave and have different sensitivities with respect to noise caused by a body movement, compared to other configurations.

In the aspect of the invention described above, it is preferable that the determined pressure is from 1 kPa to 6 kPa.

According to this configuration, the risk is low of inflicting pain on a human compared to other configurations even when measuring, for example, a pulse wave of a human.

In the aspect of the invention described above, it is preferable that the determined pressure is 2 kPa to 3 kPa.

According to this configuration, the risk is low of inflicting pain on a living body compared to other configurations even when continuously measuring a pulse wave over a comparatively long period of time, for example, for several days.

In the aspect of the invention described above, it is preferable that the determined pressure is 5 kPa to 6 kPa.

According to this configuration, it is easy to suppress occurrence of the influence of the noise caused by the body movement with respect to the capillaries existing at a comparatively shallow position in the tissue of the living body, compared to other configurations.

In the aspect of the invention described above, it is preferable that the determined wavelength is 470 nm to 610 nm.

According to this configuration, the light to be irradiated is easily reflected from hemoglobin in the blood vessels compared to other configurations.

In the aspect of the invention described above, it is preferable that a first region from the light emitting unit to the first light receiving unit in the contact surface of the living body has a portion overlapping a second region from the light emitting unit to the second light receiving unit in the contact surface.

According to this configuration, there are fewer factors of occurrence of an error compared to a case where the first region does not overlap the second region.

In the aspect of the invention described above, it is preferable that the first region intersects with the second region on the contact surface.

According to this configuration, it is possible to make positions, at which light beams respectively received by the first light receiving unit and the second light receiving unit are reflected, closer compared to other configurations.

In the aspect of the invention described above, it is preferable that the measuring unit measures the pulse wave by removing noise caused by a body movement of the living body from any one of respective signals that represent light received by the first light receiving unit and light received by the second light receiving unit based on the differences of frequencies of the signals.

According to this configuration, it is possible to remove the noise from a plurality of signals including pulse waves without depending on the difference of a wavelength of light to be irradiated.

In the aspect of the invention described above, it is preferable that the light received by the first light receiving unit is radiated through the light emitting unit through which the light received by the second light receiving unit is radiated.

According to this configuration, it is possible to make the pulse wave measuring apparatus compact.

### BRIEF DESCRIPTION OF THE DRAWINGS

The invention will be described with reference to the accompanying drawings, wherein like numbers reference like elements.
Fig. 1 is a view showing the appearance of a pulse wave measuring apparatus.
Fig. 2 is a block diagram showing a configuration of a pulse wave measuring apparatus.
Figs. 3A and 3B are views showing arrangements of each configuration of a detection unit.
Fig. 4 is a view illustrating an influence of the distance between a light emitting unit and a light receiving unit on a penetration depth of light.
Fig. 5 is a view showing a relationship between a position from which light is reflected and a signal intensity ratio.
Figs. 6A and 6B are views showing changes of light absorbance and a body movement noise sensitivity with respect to a pressure force.
Fig. 7 is a view showing a functional configuration of a control unit.
Figs. 8A and 8B are views showing arrangements of a light emitting unit and light receiving units in a modification example.

### DESCRIPTION OF EXEMPLARY EMBODIMENTS

### 1. Embodiment

### 1-1. Overall Configuration

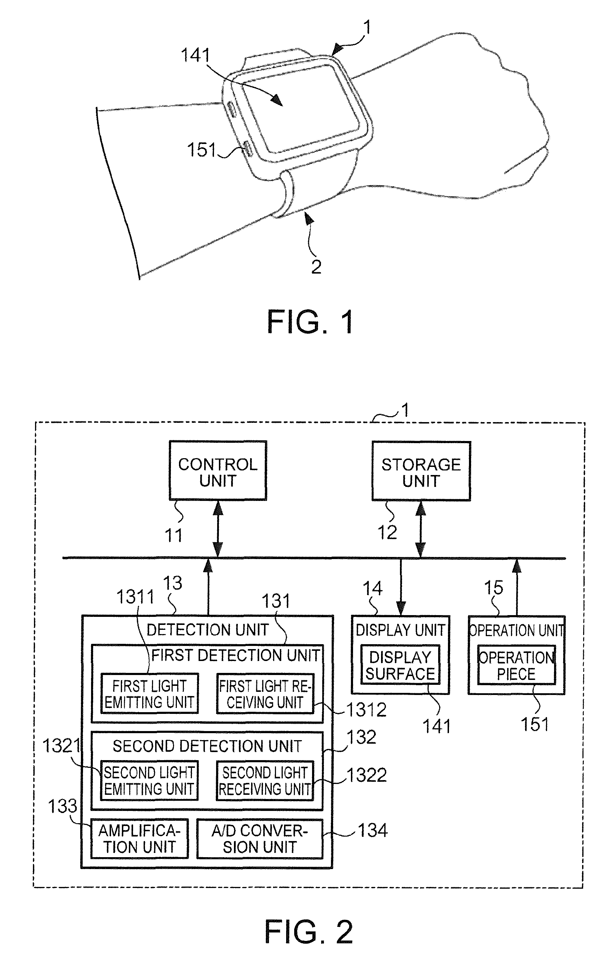

Fig. 1 is a view showing the appearance of a pulse wave measuring apparatus 1. The pulse wave measuring apparatus 1 has a configuration such as a watch which is fixed on the wrist of a user using a wrist band 2. A front surface of the pulse wave measuring apparatus 1 is provided with a display surface 141 (to be described later) formed of a liquid crystal panel and the like and a side surface of the pulse wave measuring apparatus 1 is provided with an operation piece 151 (to be described later) such as a button switch which is pressed by the user for operation. A back surface of the pulse wave measuring apparatus 1 comes into contact with the wrist of the user.

Fig. 2 is a block diagram showing a configuration of the pulse wave measuring apparatus 1. A control unit 11 has a central processing unit (CPU), a read only memory (ROM), and a random access memory (RAM), and controls each unit of the pulse wave measuring apparatus 1 by the CPU executing to read a computer program (hereinafter, simply referred to as program) stored in the ROM or a storage unit 12.

The storage unit 12 is large capacity storage means such as a solid state drive (SSD) and stores the program read by the CPU. A display unit 14 includes the display surface 141 where a liquid crystal or the like is used and causes the display surface 141 to display an image according to an instruction from the control unit 11.

An operation unit 15 includes an operation piece 151 such as a button switch for performing various instructions and receives an operation from a user to supply a signal corresponding to the operation content to the control unit 11. The operation piece 151 may include a transparent touch panel which is overlapped with the display surface 141.

A detection unit 13 has a first detection unit 131, a second detection unit 132, an amplification unit 133, and an A/D conversion unit 134. The first detection unit 131 is configured to measure a pulse wave of a living body and to output a first signal that represents the pulse wave. Specifically, the first detection unit 131 has a first light emitting unit 1311 and a first light receiving unit 1312 which are disposed so as to come into contact with the living body (in this case, a skin face of the wrist). The first light emitting unit 1311 is an example of a light source that comes into contact with the living body and irradiates the inside of the living body with light having a determined wavelength.

The second detection unit 132 is configured to measure a pulse wave of a living body at a sensitivity different from the first detection unit 131 and to output a second signal that represents the pulse wave. Here, the "sensitivity" is a noise sensitivity and represents a ratio of a noise component (intensity of a signal caused by noise) to a pulse wave component (intensity of a signal caused by a pulse wave). Specifically, the second detection unit 132 has a second light emitting unit 1321 and a second light receiving unit 1322 which are disposed so as to come into contact with the living body. The second light emitting unit 1321 is an example of a light source that comes into contact with the living body and irradiates the inside of the living body with light having a determined wavelength. The difference of the sensitivities between the above-described first detection unit 131 and the second detection unit 132 is derived from the difference between the distance between the first light emitting unit 1311 and the first light receiving unit 1312 and the distance between the second light emitting unit 1321 and the second light receiving unit 1322.

The amplification unit 133 is an amplifier that amplifies signals which are respectively output from the first detection unit 131 and the second detection unit 132. The A/D conversion unit 134 converts an analog signal amplified by the amplification unit 133 into a digital signal.

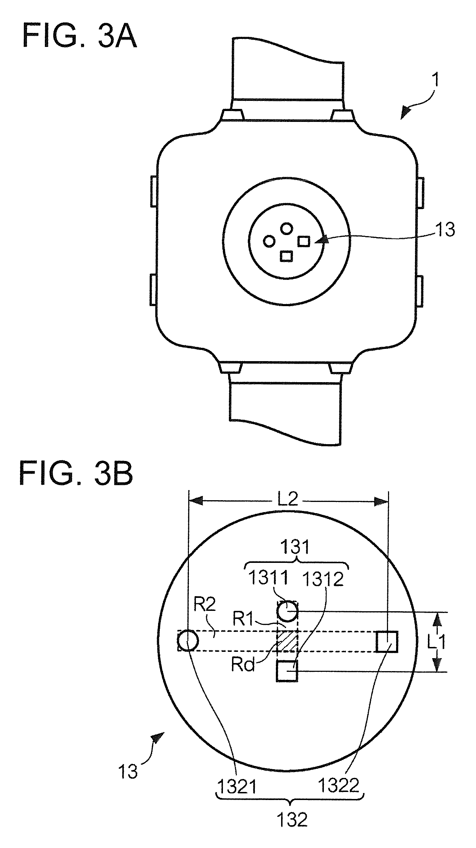

Figs. 3A and 3B are views showing arrangements of each configuration of the detection unit 13. As shown in Fig. 3A, in the pulse wave measuring apparatus 1, the detection unit 13 is disposed on a back surface (that is, a surface opposite to a surface on which the display surface 141 of the display unit 14 is provided) so as to come into contact with the skin face of the wrist of the user.

As shown in Fig. 3B, the site where the detection unit 13 comes into contact with the skin face of the wrist of the user is provided with the first light emitting unit 1311 and the first light receiving unit 1312 of the first detection unit 131 and the second light emitting unit 1321 and the second light receiving unit 1322 of the second detection unit 132. Each configuration is disposed such that the distance between the first light emitting unit 1311 and the first light receiving unit 1312 is shorter than the distance between the second light emitting unit 1321 and the second light receiving unit 1322.

In the detection unit 13, the first light receiving unit 1312 is disposed so as to be separated at a first distance L1 from the first light emitting unit 1311 along a contact surface (here, the skin face of the wrist of the user) of the living body. The first light receiving unit 1312 presses a site (hereinafter, referred to as first site) corresponding to the wrist of the user at a determined pressure and receives light, which is radiated from the first light emitting unit 1311 and is reflected from the inside of the living body, through the first site.

In addition, the second light receiving unit 1322 is disposed so as to be separated at a second distance L2 that is longer than the first distance L1 from the second light emitting unit 1321 along the contact surface of the living body. The second light receiving unit 1322 presses a site corresponding to the wrist of the user, that is, a second site which is different from the first site in the living body at the above-described pressure and receives the light reflected from the living body through the second site.

A first region R1 from the first light emitting unit 1311 and the first light receiving unit 1312 in the contact surface of the living body intersects with a second region R2 from the second light emitting unit 1321 to the second light receiving unit 1322 in the contact surface and has an overlapped region Rd as a portion where the first region R1 and the second region R2 are overlapped with each other. As such, that there is an overlapped portion in respective light passages of the first detection unit 131 and the second detection unit 132 means that each light passage permeates a common portion of the living body. Accordingly, all of the first detection unit 131 and the second detection unit 132 detect signals including pulse waves in the common portion, and therefore, there are fewer factors of occurrence of an error compared to a case where the first region R1 does not have any portion in common with the second region R2. In particular, because the first region R1 intersects with the second region R2, positions at which light beams respectively received by the first light receiving unit 1312 and the second light receiving unit 1322 are reflected become closer.

The first light emitting unit 1311 irradiates the tissue of the living body with light which has a predetermined wavelength and has an amount of light corresponding to an electric current supplied from a power source (not shown). The determined wavelength is, for example, 470 nm to 610 nm, and more preferably, 520 nm to 570 nm. The determined wavelength has a characteristic that it is easily reflected from hemoglobin in the blood vessels compared to other wavelengths. The first light receiving unit 1312 receives light which is reflected from the tissue of the living body among light beams radiated from the first light emitting unit 1311 and outputs a signal corresponding to the light receiving intensity as a first signal. Such a reflected light includes various elements. Among them, the light reflected from the hemoglobin in the blood vessels represents the pulse wave. On the other hand, for example, the reflected light is influenced by a movement (body movement) of a body of the user, and thus, there is a case where the reflected light includes noise which is irrelevant to the pulse wave.

The second light emitting unit 1321 irradiates the tissue of the living body with light which has a wavelength as the same as that of the light radiated from the first light emitting unit 1311 and has an amount of light corresponding to the supplied electric current. The second light receiving unit 1322 receives light reflected from the tissue of the living body among light beams radiated from the second light emitting unit 1321 and outputs a signal corresponding to the light receiving intensity as a second signal.

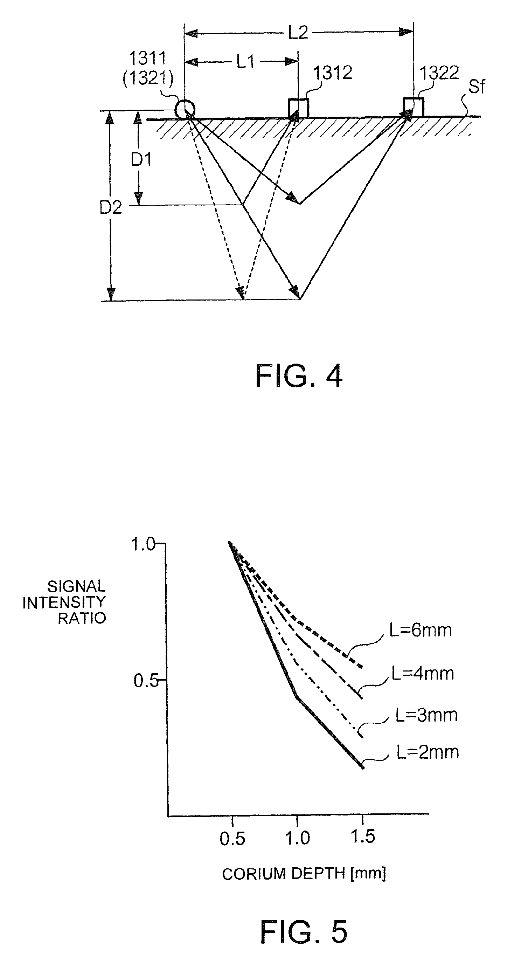

Fig. 4 is a view illustrating an influence of the distance between a light emitting unit and a light receiving unit on a penetration depth of light. A skin face Sf of the wrist of the user comes into contact with the first light emitting unit 1311, the first light receiving unit 1312, the second light emitting unit 1321, and the second light receiving unit 1322. In Fig. 4, the first light emitting unit 1311 also serves as the second light emitting unit 1321.

It is found that the shorter the distance between the light emitting unit and the light receiving unit is, the lower the relative sensitivity with respect to a deep portion of the inside of the living body is, compared to the sensitivity with respect to a shallow portion. That is, the intensity of light radiated from the first light emitting unit 1311, the light being reflected at a position having a depth D1 of the inside of the tissue of the living body and reaching the first light receiving unit 1312, is strong compared to the intensity of light which is reflected at a position having a depth D2 deeper than the depth D1 and reaches the first light receiving unit 1312. Meanwhile, the intensity of light radiated from the second light emitting unit 1321, the light being reflected at the position having the depth D1 and reaching the second light receiving unit 1322, is strong compared to the intensity of light which is reflected at the position having the depth D2 and reaches the second light receiving unit 1322. However, the difference thereof is not as much as that which occurred in the first detection unit 131. For this reason, the first detection unit 131 is configured to be suitable for detecting light reflected at a site comparatively shallow to that of the second detection unit 132.

Fig. 5 is a view showing a relationship between a position from which light is reflected and a signal intensity ratio. Fig. 5 represents ratio of the signal intensity of light beams respectively reflected from positions having 1.0 mm of a depth of the corium and 1.5 mm of a depth of the corium, with respect to the signal intensity of light which is reflected at a position having 0.5 mm of a depth of the corium and is received by the light receiving unit. For example, in a case where the distance L from the light emitting unit to the light receiving unit is 2 mm, the signal intensity of light reflected at the position having 1.5 mm of the depth of the corium is about 20% of the signal intensity of light reflected at the position having 0.5 mm of the depth of the corium, which is low. However, in a case where the distance L is 6 mm, the signal intensity thereof maintains about 55%. That is, the longer the distance L is, the easier the detection of the reflected light by the detection unit 13 at a relatively deeper position is. Therefore, the differences of the sensitivities with respect to light beams respectively reflected from a shallow position and a deep position are reduced.

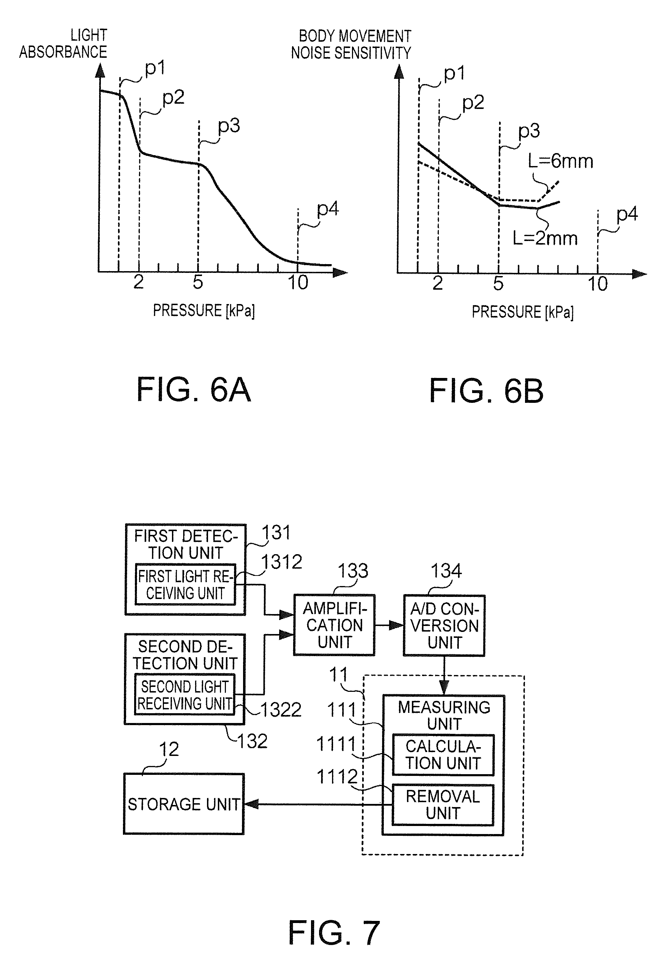

Figs. 6A and 6B are views showing changes of light absorbance and body movement noise sensitivity with respect to a pressure force. If the pressure force on the contact surface is changed by the detection unit 13, the amount or the area where the tissue of the living body is pressed changes. In particular, the dermis layer has many capillaries and is a soft tissue, and therefore, is easily pressed at a low pressure. The capillary relaxation pressure p1 (kPa) shown in Fig. 6A is a critical pressure from which the capillaries start to be closed when the pressure force exceeds p1 and the capillary closure pressure p2 (kPa) shown in Fig. 6A is a critical pressure at which the capillaries are closed when the pressure force exceeds p2. In addition, when the pressure force exceeds the artery relaxation pressure p3 (kPa) shown in Fig. 6A, the arteries start to be closed, and when the pressure force exceeds the artery closure pressure p4 (kPa), the arteries are closed.

If the pressure force, at which the detection unit 13 of the pulse wave measuring apparatus 1 presses the surface coming into contact with the living body such as a human body of a user, is set within a range of the capillary relaxation pressure p1 to the capillary closure pressure p2, the capillaries enter a state of not being closed completely. Accordingly, in this case, blood flowing in the capillaries is easily moved by the body movement. Therefore, light, which is reflected from the capillaries existing at a comparatively shallow position in the tissue of the living body, tends to include noise. As a result, the reflected light in the shallow position is easily detected in a configuration in which the distance L from the light emitting unit to the light receiving unit is 2 mm, compared to a configuration in which the distance L is 6 mm which is longer than 2 mm. Thus, the sensitivity with respect to the noise caused by the body movement (that is, body movement noise sensitivity) is high in the configuration in which the distance L is 2 mm.

In contrast, if the pressure force is set within a range of the artery relaxation pressure p3 to the artery closure pressure p4 , since the artery relaxation pressure p3 is a higher pressure than the capillary closure pressure p2, the capillaries enter a state of being closed completely and the arteries enter a state of not being closed completely. Accordingly, in this case, blood inside of the capillaries hardly moves since the capillaries are pressed. That is, light reflected from the tissue of the living body at a comparatively shallow position at which the capillaries exist hardly includes noise. However, since the arteries are not completely closed, blood inside of the arteries easily moves. That is, light reflected from the tissue of the living body at a relatively deep position at which the arteries exist tends to include noise. As a result, the reflected light in the deep position is easily detected in the configuration with 6 mm of the distance L compared to the configuration with 2 mm of the distance L. Thus, the body movement noise sensitivity is high in the configuration with 6 mm of the distance L.

As such, the body movement noise sensitivity of the first detection unit 131 and the body movement noise sensitivity of the second detection unit 132 are reversed to each other depending on what range the pressure force is set to. For example, in the example shown in Figs. 6A and 6B, if the pressure force at which the contact surface is pressed is set to a pressure within a range of 2 kPa to 3 kPa, the body movement noise sensitivity of the first detection unit 131 having a short distance from the light emitting unit to the light receiving unit becomes higher than that of the second detection unit 132. It is considered that it is because the arteries are released and the capillaries tend to include more body movement noise than the arteries since the pressure within the range of 2 kPa to 3 kPa is lower than that of the artery relaxation pressure p3. Since the pressure in the range of 2 kPa to 3 kPa is relatively low, the risk is low of inflicting pain on the living body compared to other configurations even when continuously measuring the pulse wave over a comparatively long period of time, for example, for several days.

In contrast, if the pressure force at which the contact surface is pressed is set to a pressure within a range of 5 kPa to 6 kPa, the body movement noise sensitivity of the second detection unit 132 having a long distance from the light emitting unit to the light receiving unit becomes higher than that of the first detection unit 131. The program stored in the storage unit 12 is written so that the control unit 11 specifies which body movement noise sensitivity between the first detection unit 131 and the second detection unit 132 is high depending on the set pressure.

### 1-2. Functional Configuration of Control Unit

Fig. 7 is a view showing a functional configuration of the control unit 11. The first signal output from the first light receiving unit 1312 of the first detection unit 131 and the second signal output from the second light receiving unit 1322 of the second detection unit 132 are respectively amplified via the amplification unit 133, converted into a digital signal by the A/D conversion unit 134, and then, supplied to the control unit 11. That is, the control unit 11 functions as a first acquisition unit that acquires the first signal, which represents a pulse wave of a living body, from the first detection unit 131 that measures the pulse wave. In addition, the control unit 11 functions as a second acquisition unit that acquires the second signal, which represents the pulse wave of the living body, from the second detection unit 132 that measures the pulse wave at a sensitivity different from the first detection unit 131.

The control unit 11 that acquired the first signal and the second signal functions as a measuring unit 111 that measures the pulse wave of the living body based on each of the signals. The measuring unit 111 has a calculation unit 1111 that calculates the differences of frequencies of the first signal and the second signal and a removal unit 1112 that removes noise caused by the body movement of the living body from any one of the first signal and the second signal based on the differences of the frequencies calculated by the calculation unit 1111. The measuring unit 111 measures the pulse wave of the living body by extracting body movement noise from the signal output from a detection unit having a high body movement noise sensitivity and by removing the body movement noise from the signal output from a detection unit having a low body movement noise sensitivity. The numerical value or the like that represents the measured pulse wave is stored in the storage unit 12.

As described above, in the two detection units having light emitting units respectively radiating light beams having the same wavelength, the pulse wave measuring apparatus 1 can measure the pulse wave with high accuracy compared to the related art since noise included in the signals output therefrom is removed using the different distances from the light emitting units to the light receiving units.

### 2. Modification Example

An embodiment has been described above, but the content of the embodiment can be modified as in the following. In addition, the following modification examples may also be combined together.

### 2-1. Modification Example 1

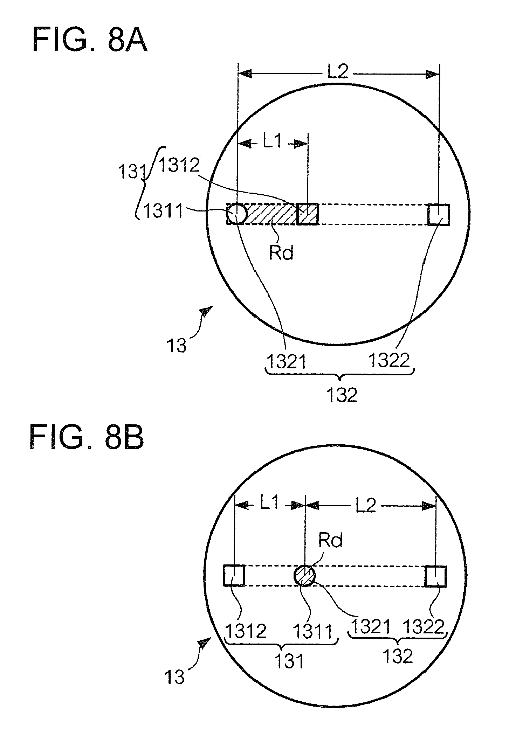

Although the first region R1 intersects the second region R2 in the above-described embodiment, the disposition of each of the regions is not limited thereto. In addition, although the first light emitting unit 1311 is formed separately from the second light emitting unit 1321 in the embodiment, the first light emitting unit 1311 may serve as the second light emitting unit 1321. Figs. 8A and 8B are views showing arrangements of a light emitting unit and light receiving units in the modification example. In the example, the first light emitting unit 1311 serves as the second light emitting unit 1321 as shown in Fig. 8A. Moreover, the second light receiving unit 1322 is provided at an end of a region extending from the first light emitting unit 1311 to the first light receiving unit 1312. That is, the first region R1 from the first light emitting unit 1311 to the first light receiving unit 1312 may be included in the second region R2 from the second light emitting unit 1321 and the second light receiving unit 1322.

### 2-2. Modification Example 2

In addition, as shown in Fig. 8B, the first light emitting unit 1311 may serve as the second light emitting unit 1321 and the second light receiving unit 1322 may be provided in an opposite side in a direction, in which the first light receiving unit 1312 is provided, when seen from the first light emitting unit 1311. In this case, the region where the first light emitting unit 1311 is provided corresponds to the overlapped region Rd.

### 2-3. Modification Example 3

In the above-described embodiment, the control unit 11 which has acquired the first signal and the second signal measures the pulse wave of the living body by calculating the differences of the frequencies of each of the signals and removing the noise caused by the body movement of the living body from any one of the first signal and the second signal based on the differences of the frequencies. However, means for removing the noise caused by the body movement is not limited thereto. For example, the control unit 11 may calculate the differences of the intensity of each of the signals to remove the noise caused by the body movement.

### 2-4. Modification Example 4

The pressure force, at which the surface coming into contact with the living body is pressed by the detection unit 13 of the pulse wave measuring apparatus 1, is set in advance in the above-described embodiment, but the pressure force may be changed. In this case, the pulse wave measuring apparatus 1 may have a measurement part that measures the pressure force and the control unit 11 of the pulse wave measuring apparatus 1 may specify as to which body movement noise sensitivity between the first detection unit 131 and the second detection unit 132 is high depending on the pressure force measured by the measurement part.

### 2-5. Modification Example 5

The number of detection units is not limited to that described in the embodiment. Although an example in which the pulse wave measuring apparatus 1 has two detection units, which are the first detection unit 131 and the second detection unit 132, is described in the embodiment, the pulse wave measuring apparatus 1 may also have three or more detection units. In this case, the pulse wave measuring apparatus 1 may acquire signals that represent pulse waves using signals output from two detection units selected from the three or more detection units.

### 2-6. Modification Example 6

Although the range of the first distance L1 is not mentioned in the above-described embodiment, it is desirable that the first distance L1 is 2 mm to 4 mm. In addition, it is desirable that the second distance L2 is longer than the first distance L1 and be 4 mm to 10 mm.

### 2-7. Modification Example 7

The example illustrated in Figs. 6A and 6B of above-described embodiment shows that the body movement noise sensitivity of the first detection unit 131 becomes higher than that of the second detection unit 132 if the pressure force at which the contact surface is pressed is set to a pressure within the range of 2 kPa to 3 kPa, and the body movement noise sensitivity of the second detection unit 132 becomes higher than that of the first detection unit 131 if the pressure force is set to a pressure within the range of 5 kPa to 6 kPa. However, the setting of the pressure force is not limited to the range. It is desirable that the pressure force, at which the contact surface is pressed, is 1 kPa to 6 kPa from the relationship between a burden that the living body receives from the pressure and detection accuracy.

## Claims

1. A pulse wave measuring apparatus comprising:
a first light receiving unit which is disposed so as to be separated at a first distance along a contact surface of a living body from a light emitting unit and radiates light with a determined wavelength toward the inside of the living body, presses a first site of the living body at a determined pressure, and receives the light reflected from the living body through the first site;
a second light receiving unit which is disposed so as to be separated at a second distance along the contact surface of the living body that is longer than the first distance from the light emitting unit and radiates light with the wavelength toward the inside of the living body, presses a second site that is different from the first site of the living body at the pressure, and receives the light reflected from the living body through the second site; and
a measuring unit that measures the pulse wave of the living body based on respective signals that represent light received by the first light receiving unit and light received by the second light receiving unit.

2. The pulse wave measuring apparatus according to claim 1,
wherein the first distance is from 2 mm to 4 mm.

3. The pulse wave measuring apparatus according to claim 1 or 2,
wherein the second distance is from 4 mm to 10 mm.

4. The pulse wave measuring apparatus according to any one of the preceding claims,
wherein the determined pressure is from 1 kPa to 6 kPa.

5. The pulse wave measuring apparatus according to claim 4,
wherein the determined pressure is 2 kPa to 3 kPa.

6. The pulse wave measuring apparatus according to claim 4,
wherein the determined pressure is 5 kPa to 6 kPa.

7. The pulse wave measuring apparatus according to claim any one of the preceding claims ,
wherein the determined wavelength is 470 nm to 610 nm.

8. The pulse wave measuring apparatus according to any one of the preceding claims ,
wherein a first region from the light emitting unit to the first light receiving unit in the contact surface of the living body has a portion overlapping a second region from the light emitting unit to the second light receiving unit in the contact surface.

9. The pulse wave measuring apparatus according to claim 8,
wherein the first region intersects with the second region on the contact surface.

10. The pulse wave measuring apparatus according to any one of the preceding claims,
wherein the measuring unit measures the pulse wave by removing noise caused by a movement of the living body from any one of respective signals that represent light received by the first light receiving unit and light received by the second light receiving unit based on the differences of frequencies of the signals.

11. The pulse wave measuring apparatus according to any one of the preceding claims,
wherein the light received by the first light receiving unit is radiated through the light emitting unit through which the light received by the second light receiving unit is radiated.
